# EUROPÄISCHE PATENTANMELDUNG

(11) **EP 4 186 481 A1**
(43) Veröffentlichungstag der Anmeldung: **31.05.2023**
(21) Anmeldenummer: 21211495.3
(22) Anmeldetag: 30.11.2021
(51) Int. Cl.: A61F 13/02

(54) **PFLASTER ZUR AUFBRINGUNG AUF DIE HAUT**

(71) Anmelder: Barth, Steffen, 58091 Hagen (DE)
(72) Erfinder: Barth, Steffen, 58091 Hagen (DE)
(74) Vertreter: Brunotte, Joachim Wilhelm Eberhard

(57) **Zusammenfassung**

Die Erfindung betrifft ein Pflaster zur Aufbringung auf die Haut für medizinische, kosmetische und/oder orthopädische Zwecke. Das Pflaster weist eine erste Folie (1) auf, mit einem ersten Trägermaterial, das auf zumindest einer Seite eine Metall-Beschichtung (21) aufweist. Ferner weist das Pflaster eine zweite Folie (3) auf, mit einem zweiten Trägermaterial, das auf einer Seite mit einem Klebstoff beschichtet ist, sodass an dem zweiten Trägermaterial eine Klebstoffschicht (23) gebildet ist. Die erste Folie (1) ist über die Klebstoffschicht (23) mit der zweiten Folie (3) verbunden, sodass eine zweilagige Verbundfolie gebildet ist, bei der eine außenseitig der Verbundfolie liegende Oberfläche durch die Metall-Beschichtung (21) oder eine der Metall-Beschichtungen der ersten Folie (1) gebildet ist. Die erste Folie weist eine kleinere Breite und eine kleinere Länge als die zweite Folie auf und ist derart an der zweiten Folie positioniert, dass Klebstoff der Klebstoffschicht (23) auf der der ersten Folie zugewandten Seite der zweiten Folie (3) umlaufend um die erste Folie eine Oberfläche der Verbundfolie bildet.

## Beschreibung

Die Erfindung betrifft ein Pflaster zur Aufbringung auf die Haut für medizinische, kosmetische und/oder orthopädische Zwecke.

Pflaster sind in der Medizin und der Kosmetik in verschiedenen Ausprägungen bekannt. Häufig werden Pflaster zur transdermalen Verabreichung kontrollierter Wirkstoffmengen verwendet. Bei solchen Pflastern kann beispielsweise der Wirkstoff in einem Polymermatrixfilm gelöst oder suspendiert sein, durch den der Wirkstoff in die Haut diffundiert (vgl. US 4 839 174).

Die bekannten Pflaster sind nur bedingt zur Behandlung entzündlicher Bereiche geeignet. Insbesondere innere Entzündungen, beispielsweise an Gelenken mit Kapselverletzung oder der so genannte Tennisarm, werden in der Regel durch Stilllegung mittels Bandage und gegebenenfalls mit medikamentöser Unterstützung behandelt. Derartige Behandlungen sind langwierig und können, insbesondere im Fall medikamentöser Behandlung, zu erheblichen Beeinträchtigungen des Körpers führen.

Zur Verbesserung der Behandlung innerer Entzündungen ist aus der DE 103 06 187 A1 ein Pflaster bekannt, welches aus einem Klebestreifen besteht, auf dem eine elektrisch leitfähige Schicht aufgeklebt ist. Die Schicht ist derart auf dem Klebestreifen angeordnet, dass der umlaufende äußere Rand des Klebestreifens unbedeckt ist. Die unbedeckte Klebefläche dient der Fixierung des Pflasters auf der Haut.

Das bekannte Pflaster erfüllt die an es gestellten Anforderungen. Abhängig von der Wahl der elektrisch leitfähigen Schicht ist es jedoch auf einen relativ engen Wirkungsbereich, z.B. entzündliche Vorgänge in einem Gelenk, begrenzt. Auch ist durch die Verwendung eines Klebestreifens, auf den dann die beschichtete Folie aufgebracht ist, eine Dicke des Pflasters hervorgerufen, die bei verschiedenen Anwendungen negative Einflüsse auf den Tragekomfort hat. Dies tritt beispielsweise bei der Behandlung von Kapselentzündungen im Bereich des Fußes, insbesondere im Bereich der Zehen, auf, da die Füße in der Regel im Schuhwerk untergebracht sind.

Bei sportlichen Aktivitäten handelt es sich hierbei meist um eng anliegende Schuhe, die dem Träger ein gutes Gefühl für den Kontakt mit dem Boden vermitteln sollen. Darüber hinaus sind beispielsweise Damen-Pumps ebenfalls sehr eng geschnitten. Bei diesen oder ähnlichen Anwendungen sind die bekannten Pflaster nicht ohne Komforteinbußen an den Zehen anbringbar, da durch die Dicke der bekannten Pflaster eine Druckstelle entsteht, die sich genau auf der zu behandelnden Stelle befindet. Durch die Druckstellen sind die Behandlungsergebnisse negativ beeinflusst; im äußersten Fall kann es an den Druckstellen zu Rötungen bis hin zu Hautaufschürfungen kommen. Auch beim Tragen von Kompressionsstrümpfen ergeben sich die genannten Probleme.

Hinzu kommt, dass die bekannten Pflaster das Anziehen der sehr engen Kompressionsstrümpfe behindern bzw. die Pflaster beim Anziehen abgelöst werden.

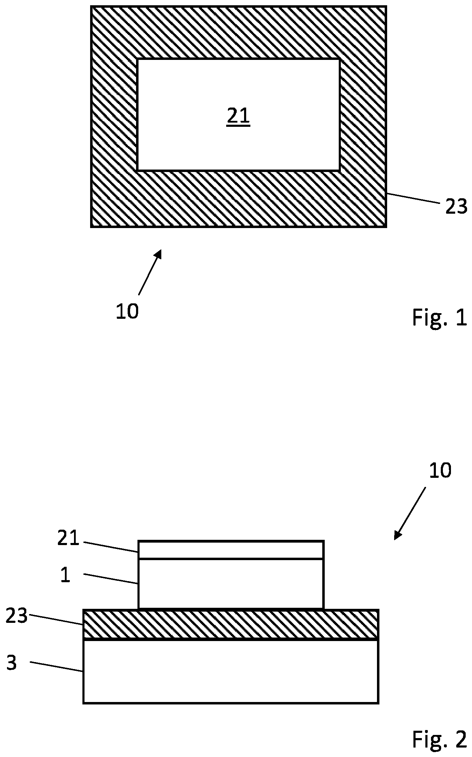

DE 1 798 343 U offenbart einen Verband, insbesondere eine Binde oder ein mit Klebstoff versehendes Pflaster. Der Verband weist zwei durch eine Zwischenlage voneinander getrennte Metallfolien auf, die einem mit Klebstoff versehenden Pflaster zugeordnet sein können. Die eine Folie kann aus einem anderen Werkstoff bestehen als die andere. Als äußere Schicht, die auf den zu behandelnden Körperteil aufgelegt wird, empfiehlt sich eine Folie, die aus einem Edelmetall besteht oder ein solches enthält, beispielsweise Silber, Gold oder dergleichen. Als Ausführungsform wird Kondensatorpapier beschrieben, das aus mehreren, durch Papierlagen getrennte Silberpapieren besteht. Figur 1 zeigt, dass sich eine mit Klebstoff versehene Fläche eines solchen Pflasters umlaufend um das Kondensatorpapier herum erstrecken kann.

US 2,755,800 beschreibt eine klebende Bandage, die eine Metallfolie aufweist, z. B. eine Aluminiumfolie, die auf beiden Seiten auf einem geeigneten flexiblen Pflaster befestigt ist, welches aus Plastik gebildet sein kann. Auf dem Pflaster befindet sich eine Klebstoffbeschichtung als Unterschicht. Randbereiche und die Folie tendieren dazu, eine im Wesentlichen luft- und wasserdichte Abdichtung um die Haut-Verhärtung zu bilden.

WO 2009/106323 A1 offenbart ein Pflaster zur Aufbringung auf die Haut für medizinische, kosmetische und/oder orthopädische Zwecke, wobei das Pflaster eine Folie aufweist, die aus einem Trägermaterial besteht, das auf beiden Seiten mit einer therapeutisch wirksamen Beschichtung versehen ist. An den umlaufenden Rändern der Folie und/oder außerhalb der umlaufenden Ränder der Folie ist umlaufend Klebstoff vorgesehen, so dass durch Aufkleben des Pflasters auf die Haut eines Patienten der von der Folie abgedeckte Hautbereich gegen die Luft in der Umgebung abgeschlossen wird. Bei der Beschichtung auf einer Seite handelt es sich um eine Silber-Beschichtung oder silberfarbene Metallbeschichtung. Bei der Beschichtung auf der gegenüberliegenden Seite handelt es sich um eine Gold-Beschichtung oder goldfarbene Metallbeschichtung. Die Folie einschließlich beider Beschichtungen hat eine Dicke von weniger als 100 µm.

Auch dieses Pflaster erfüllt seinen Zweck als Pflaster für medizinische, kosmetische und/oder orthopädische Zwecke. Es ist in WO 2009/106323 A1 auch eine Variante mit einer zusätzlichen zweiten Trägerschicht offenbart. Auf diese Weise kann das Pflaster selbst sehr dünn ausgeführt werden. Die zweite Trägerschicht ist z.B. eine vollflächig mit Klebstoff versehene Gewebeschicht oder andere Schicht, wie sie üblicherweise bei Tapeverbänden verwendet wird.

Der Erfindung liegt die Aufgabe zugrunde, ein Pflaster zu schaffen, welches die guten Behandlungsergebnisse der aus dem Stand der Technik bekannten Pflaster verbessert und den Anwendungsbereich erweitert. Insbesondere soll das Pflaster eine Diagnose der biochemischen Situation auf der Haut des Patienten erleichtern.

Es wird ein Pflaster zur Aufbringung auf die Haut für medizinische, kosmetische und/oder orthopädische Zwecke vorgeschlagen, wobei
- das Pflaster eine erste Folie aufweist, mit einem ersten Trägermaterial, das auf zumindest einer Seite eine Metall-Beschichtung aufweist,
- das Pflaster eine zweite Folie aufweist, mit einem zweiten Trägermaterial, das auf einer Seite mit einem Klebstoff beschichtet ist, sodass an dem zweiten Trägermaterial eine Klebstoffschicht gebildet ist,
- die erste Folie über die Klebstoffschicht mit der zweiten Folie verbunden ist, sodass eine zweilagige Verbundfolie gebildet ist, bei der eine außenseitig der Verbundfolie liegende Oberfläche durch die Metall-Beschichtung oder eine der Metall-Beschichtungen der ersten Folie gebildet ist, und
- die erste Folie eine kleinere Breite und eine kleinere Länge als die zweite Folie aufweist und derart an der zweiten Folie positioniert ist, dass Klebstoff der Klebstoffschicht auf der der ersten Folie zugewandten Seite der zweiten Folie umlaufend um die erste Folie eine Oberfläche der Verbundfolie bildet.

Ferner wird ein Verfahren vorgeschlagen zur Herstellung eines Pflasters zur Aufbringung auf die Haut für medizinische, kosmetische und/oder orthopädische Zwecke, insbesondere zur Herstellung des Pflasters in einer der in dieser Beschreibung beschriebenen Ausgestaltungen, wobei
- eine erste Folie bereitgestellt wird, mit einem ersten Trägermaterial, das auf zumindest einer Seite eine Metall-Beschichtung aufweist,
- eine zweite Folie bereitgestellt wird, mit einem zweiten Trägermaterial, das auf einer Seite mit einem Klebstoff beschichtet ist, sodass an dem zweiten Trägermaterial eine Klebstoffschicht gebildet ist,
- die erste Folie über die Klebstoffschicht mit der zweiten Folie verbunden wird, sodass eine zweilagige Verbundfolie gebildet wird, bei der eine außenseitig der Verbundfolie liegende Oberfläche durch die Metall-Beschichtung oder eine der Metall-Beschichtungen der ersten Folie gebildet wird, und
- die erste Folie eine kleinere Breite und eine kleinere Länge als die zweite Folie aufweist und derart an der zweiten Folie positioniert wird, dass Klebstoff der Klebstoffschicht auf der der ersten Folie zugewandten Seite der zweiten Folie umlaufend um die erste Folie eine Oberfläche der Verbundfolie bildet.

Der Duden definiert den Begriff "Folie" als "sehr dünnes Material zum Bekleben oder Verpacken". Dies bedeutet, dass die Folie eine sehr viel größere Länge und sehr viel größere Breite als ihre Dicke hat. Auf bevorzugte Abmessungen der Dicke wird noch eingegangen. Insbesondere kann die Dicke wie bei Folien üblich konstant sein, d. h. in jedem Bereich der Folie gleich sein, bei den üblichen herstellungsbedingten Schwankungen. Absichtliche vorhandene Schwankungen der Dicke wie zum Beispiel bei einer Folie mit strukturierter Oberfläche werden in Bezug auf die vorliegende Erfindung nicht bevorzugt.

Die zweilagige Verbundfolie kann daher wie die oben genannte Variante des aus WO 2009/106323 A1 bekannten Pflasters auf die Haut einer Person aufgebracht werden und dabei über den sich um die erste Folie herum erstreckenden Klebstoff an der Haut festgeklebt werden. Dabei kann ein zu einem hohen Grad luftdichter Abschluss der unterhalb der ersten Folie liegenden Hautpartie erreicht werden, denn die Folie selbst ist vorzugsweise aus Kunststoff gefertigt und luftundurchlässig. Ferner ist der Klebstoff der Klebstoffschicht, welcher die um die erste Folie umlaufende Oberfläche bildet, vorzugsweise vollflächig vorgesehen, d. h. ohne inselartige Bereiche oder Randbereiche, in denen auf der der ersten Folie zugewandten Oberfläche der zweiten Folie kein Klebstoff vorhanden ist.

Gegenüber dem aus WO 2009/106323 A1 bekannten Pflaster bildet die zweite Folie die zweite Trägerschicht. Somit kann das vollständig oder überwiegend aus der zweilagigen Verbundfolie bestehende Pflaster sehr dünn ausgeführt sein. In Bezug auf die oben genannten Applikationen unter der Kleidung, an den Beinen oder an den Füssen oder zum Beispiel unter einem Verband wird der Tragekomfort erhöht.

Wenn das Pflaster nicht vollständig aus der zweilagigen Verbundfolie mit den genannten Beschichtungen besteht, dann ist z. B. lediglich noch ein Aufdruck (oder ein anderer insbesondere örtlich partieller Materialauftrag) auf die nicht mit der Klebstoffschicht versehene Oberfläche der zweiten Folie und/oder eine Schutzschicht auf der Seite der Klebstoffschicht vorhanden. Die Schutzschicht kann insbesondere verhindern, dass Gegenstände unbeabsichtigt an den Klebstoff gelangen. Vor dem Applizieren des Pflasters auf der Haut wird die Schutzschicht entfernt. Die Schutzschicht kann insbesondere aus einem durchgehend festen und vorzugsweise formstabilen Material bestehen, sodass die Schutzschicht auf einfache Weise von der Klebstoffschicht abgezogen werden kann.

Das Trägermaterial zumindest einer der beiden Folien kann z. B. ein natürliches oder künstliches Polymer sein, vorzugsweise ein Polyolefin, z. B. ein Polyethylen, und/oder ein Polyester, insbesondere Polyethylenterephthalat (PET). Das Polymer kann alternativ oder zusätzlich einen oder mehrere der folgenden Stoffe aufweisen: Synthesekautschuke und Naturkautschuke, Polychloroprene, Polyvinylether und Polyurethane, die mit Zusätzen wie Harzen, Weichmachern und/oder Antioxidantien kombiniert werden können.

Insbesondere kann das Polymer zumindest einen Füllstoff und/oder Farbstoff aufweisen, wie Siliziumdioxid oder Titandioxid. Insbesondere kann der Stoff mit einer Konzentration von 0,01 bis 1 Gewichtsprozent, insbesondere 0,08 bis 0,12 Gewichtsprozent in dem Trägermaterial vorhanden sein.

Die erste und/oder zweite Folie kann biaxial orientiert sein, insbesondere im Fall einer PET-Folie. Dies kann in an sich bekannter Weise durch ein Reckverfahren erreicht werden.

Insbesondere kann die Dichte der ersten und/oder zweiten Folie in einem Bereich von 1,20 bis 1,50 g/cm3, vorzugsweise in einem Bereich von 1,30 bis 1,45 g/cm3 und konkret in einem Bereich von 1,38 bis 1,40 g/cm3 liegen.

Vorzugsweise handelt es sich bei dem Klebstoff um einen Haftklebstoff. Insbesondere kann er aus der folgenden Gruppe von Stoffen ausgewählt sein: Acrylate (insbesondere Poly(meth)acrylate), Silicone und/oder Polyisobutylene insbesondere Polysiobutylene in Verbindung mit Klebharzen. Basispolymere weiterer möglicher Haftklebstoffe sind Synthesekautschuke und Naturkautschuke, Polyester, Polychloroprene, Polyvinylether und Polyurethane, die mit Zusätzen wie Harzen, Weichmachern und/oder Antioxidantien kombiniert werden können. Die Klebstoffe können als wässrige Systeme (Dispersionsklebstoffe), als lösemittelbasierende Systeme (Lösemittelklebstoffe) und als Schmelzklebstoff-Systeme realisiert werden. Insbesondere kann der Haftklebstoff ein Polyacrylat auf Basis von Acrylsäure, Butylacrylat, 2-Ethylhexylacrylat und Vinylacetat oder auf Basis von Acrylsäure, 2-Ethylhexylacrylat und Methylacrylat sein. Alternativ eine zweiphasige Klebematrix verwendet werden, die hydrophile Polyacrylate und lipophile Hilfsstoffe aufweist.

Polymermaterialien als Trägermaterial der Folie können dünn ausgeführt werden und sind für das Aufbringen auf die Haut dennoch als robustes Trägermaterial geeignet. Ferner können Polymerfolien kostengünstig bei reproduzierbare Dicke hergestellt werden. Ferner eignen sie sich für eine Herstellung ohne unerwünschte Verunreinigungen. Insbesondere können sie in einem Vakuum hergestellt und/oder beschichtet werden. Die zumindest eine Metall-Beschichtung kann zum Beispiel in einem Vakuum auf die erste Folie aufgedampft oder aufgesputtert werden.

Bei dem Material der Metall-Beschichtung kann es sich um ein einziges Metall (ein einziges chemisches Element) oder um eine Legierung oder Kombination von mehreren Metallen handeln. Es wird bevorzugt, dass die Metall-Beschichtung zumindest Aluminium aufweist. Aluminium hat den Vorteil, dass es in normaler Luftatmosphäre eine dünne Passivierungsschicht an seiner Oberfläche ausbildet und daher nicht ohne Weiteres mit Luftbestandteilen oder bei Kontakt chemisch reagiert. Wie noch näher erläutert wird, ist allerdings eine Reaktion mit dem Hautschweiß, je nach pH-Wert der Haut, erwünscht. Eine solche Reaktion kann trotz der Passivierungsschicht stattfinden.

Als elektrisch leitfähige Beschichtung kann die zumindest eine Metall-Beschichtung ferner die biochemischen Prozesse auf, in und unter der Haut beeinflussen. Je nach Wahl des Metalls kann eine therapeutische Wirkung in der Haut oder dem Körper des Patienten erzielt werden. So kann insbesondere die heilende Wirkung eines Edelmetalls auf der entfaltet werden.

Bei jeglicher Art von Metall-Beschichtung kann sich diese unter dem Einfluss der biochemischen Substanzen auf und in der Haut zumindest stellenweise auflösen. Unter "auflösen" wird auch verstanden, dass sich in der Metall-Beschichtung Risse oder Löcher bilden. Zur Auflösung kann es insbesondere dann kommen, wenn der Hautschweiß einen pH-Wert im sauren Bereich hat. Dagegen kann Hautschweiß im basischen Bereich die Trägerfolie unversehrt lassen, z.B. wenn das Trägermaterial ein Polymer (z.B. Polyester) mit weniger als 20 µm Dicke ist. Daher kann das Pflaster die Therapie abhängig von der biochemischen Situation auf der Haut des Patienten steuern. Auch dient das Pflaster daher insbesondere als Diagnosehilfe: Wenn der Hautschweiß zur Auflösung von Teilen des Pflasters führt, erlaubt dies den Schluss, dass der Hautschweiß einen pH-Wert im sauren Bereich hat und daher entzündliche Vorgänge vorliegen können.

Um die Reaktionsfläche von Hautschweiß mit der Metall-Beschichtung zu erhöhen, kann die an der Hautoberfläche anzuordnende Metall-Beschichtung optional Aussparungen aufweisen. Bevorzugt wird allerdings eine gleichmäßig dicke und vorzugsweise auch homogene Metall-Beschichtung auf der an der Hautoberfläche anzuordnenden Seite der ersten Folie. Vorteile einer solchen konstanten Dicke sind in der WO 2009/106323 A1 beschrieben.

Durch die Metall-Beschichtung(en) der ersten Folie wird die von der Haut des Patienten emittierte Wärmestrahlung in besonders hohem Maße zurückreflektiert. Es hat sich gezeigt, dass die Heilungsprozesse in den lebenden Hautschichten und darunter dadurch wesentlich gefördert werden. Dies gilt insbesondere auch deshalb, weil das Pflaster mit umlaufend um die erste Folie angeordnetem Klebstoff auf die Haut geklebt wird und dadurch kein oder lediglich ein sehr geringer Luftaustausch zwischen der von dem Pflaster abgedeckten Hautoberfläche und der Umgebung stattfinden kann. Ein solcher Luftabschluss wirkt außerdem regulierend auf einen veränderten pH-Wert des Hautschweißes. Insbesondere kann der Hautschweiß nicht oder nur in sehr geringem Maß verdunsten. Dadurch wird einerseits keine Körperwärme für die Verdunstung verbraucht und entsteht andererseits auf der von dem Pflaster abgedeckten Hautoberfläche ein Bereich mit Hautschweiß, der heilend wirkt und/oder wie bereits beschrieben zur Diagnose beiträgt. Bei einer antibakteriell wirksamen Metall-Beschichtung auf der näher an der Haut liegenden Seite der ersten Folie wird der Hautschweiß gereinigt und kann in gereinigter Form, auch insbesondere ohne Zusatz von Medikamenten, wieder in die oberen Hautschichten eindringen und den Heilungsprozess bzw. den kosmetischen Prozess fördern.

Auf Anwendungsmöglichkeiten des Pflasters, die in der WO 2009/106323 A1 beschrieben sind, insbesondere zur Behandlung von Druckstellen, auch wenn sich Blasen gebildet haben, zur Narbenbehandlung, als orthopädisches Pflaster, gemäß der traditionellen chinesischen Medizin (TCM), zur Reduktion der Auffälligkeit von Hautfalten, zur Steigerung des Durchflusses durch Venen und/oder Lymphgefäße und zur Diagnose wird verwiesen. Die gegenüber der in WO 2009/106323 A1 offenbarten Pflaster mit umlaufenden Klebstoffrand geringere Dicke erhöht dabei den möglichen Anwendungsbereich und/oder reduziert die möglichen Nachteile und Nebenwirkungen aufgrund einer relevanten Pflasterdicke.

Wie erwähnt kann die Metall-Beschichtung, welche die außenseitig der Verbundfolie liegende Oberfläche bildet, Aluminium aufweisen oder daraus bestehen. Andere alternativ oder zusätzlich für die Metall-Beschichtung in Frage kommende Metalle sind Gold oder goldfarbene Stoffe (z.B. Messing oder eloxiertes Aluminium, das entsprechend gefärbt wurde und als so genanntes Goldeloxal bekannt ist) sowie Silber oder silberfarbene Stoffe.

Die Dicke der ersten und/oder der zweiten Folie (Trägermaterial und Beschichtungen) beträgt insbesondere jeweils weniger als 100µm, vorzugsweise weniger als 20 µm und liegt z.B. im Bereich von 8 bis 12µm. Insbesondere können sich die Dicken der ersten Folie und der zweiten Folie voneinander unterscheiden. Zum Beispiel wenn bei der zu erwartenden mechanischen Belastung des Pflasters eine größere mechanische Robustheit gegen Beschädigung oder Zerstörung gewünscht ist, kann die Dicke der zweiten Folie größer sein als die Dicke der ersten Folie. Dies ermöglicht es insbesondere, für verschiedene Ausführungsformen des Pflasters immer dieselbe Ausführungsform der ersten Folie (zum Beispiel mit derselben Dicke und optional auch derselben Art von Metall-Beschichtung) zu wählen, diese erste Folie aber mit unterschiedlich dicken zweiten Folien zu der zweilagigen Verbundfolie zu kombinieren.

Somit ist ein Pflaster geschaffen, das die guten Behandlungsergebnisse der insbesondere aus WO 2009/106323 A1 bekannten Pflaster beinhaltet, gleichzeitig jedoch aufgrund der noch dünnen Ausführung keine Behinderung des Tragekomforts bzw. der Handhabung hervorruft. Insbesondere wird Tragekomfort beziehungsweise die Anwendbarkeit auch in engem Schuhwerk oder Kompressionsstrümpfen ermöglicht.

Weiterbildungen und Ausgestaltungen der Erfindung sind in den Unteransprüchen angegeben.

Gemäß einer Ausgestaltung hat die erste Folie einschließlich der Metall-Beschichtung(en) eine Dicke von weniger als 20 µm. Auf Vorteile hiervon wurde bereits eingegangen. Insbesondere reicht eine Dicke von weniger als 10 µm im Fall eines Polymer (insbesondere eines Polyethylens) als Trägermaterial für die erste Folie aus, vor allem da noch die zweite Folie vorhanden ist und die erste Folie vollflächig über die Klebstoffschicht mit der zweiten Folie verbunden ist. Der Verbund ist besonders robust und aufgrund der üblicherweise aus verformbarem Klebstoff bestehenden Klebstoffschicht dennoch nicht starr an eine einzige Form gebunden. Vielmehr erlaubt der Verbund das Krümmen des Pflasters.

Alternativ oder zusätzlich kann das Trägermaterial der ersten Folie und/oder der zweiten Folie durchsichtig sein. Insbesondere wenn das Trägermaterial beider Folien durchsichtig ist, kann von der nach Applikation auf die Haut außen (von der Haut entfernt) liegenden Seite aus die Metall-Beschichtung auf der Hautseite des Pflasters gesehen werden. Das Ergebnis der oben beschriebenen Prozesse, die zu einer Veränderung der Metall-Beschichtung führen, können daher durch den zweilagigen Verbund der beiden Folien hindurch betrachtet werden, wodurch eine Diagnose wiederholt möglich sowie erleichtert wird und das Pflaster nicht zur Betrachtung der veränderten Metall-Beschichtung von der Haut entfernt werden muss. Wenn beide Trägermaterialien durchsichtig sind, wird es daher bevorzugt, dass sich auf der Seite der ersten Folie, die zur zweiten Folie weist, keine Metall-Beschichtung befindet. Die erste Folie weist in diesem Fall daher lediglich eine einzige Metall-Beschichtung auf, welche bei Applikation des Pflasters die zur Haut weisende Oberfläche bildet.

Die Metall-Beschichtung, welche die außenseitig der Verbundfolie liegende Oberfläche bildet, ist insbesondere undurchsichtig. Dies setzt eine entsprechende Schichtdicke der Metall-Beschichtung von deutlich mehr als 100 nm voraus, insbesondere von mehr als 500 nm.

Hierdurch wird die Reflexion von Wärmestrahlung der Haut zurück Richtung Haut gesteigert und wird die Diagnose bei saurem pH-Wert des Hautschweißes erleichtert. Wenn sich die Metall-Beschichtung stellenweise auflöst, dann kann an diesen Stellen Licht hindurchdringen.

Die genannten Ausgestaltungen beziehen sich nicht nur auf das Pflaster sondern auch auf Ausgestaltungen des Verfahrens zur Herstellung des Pflasters.

Bei einer Ausgestaltung des Herstellungsverfahrens wird die erste Folie durch Druck auf die Metall-Beschichtung, welche die außenseitig der Verbundfolie liegende Oberfläche bilden soll, an die Klebstoffschicht der zweiten Folie angepresst, um die Verbundfolie zu bilden. Durch den Druck wird die erste Folie an die Klebstoffschicht der zweiten Folie angepasst, wodurch der Folienverbund hergestellt wird. Insbesondere kann die der Klebstoffschicht abgewandte Seite der zweiten Folie vollflächig an einem Festkörper mit insbesondere ebener Oberfläche anliegen, während der Druck ausgeübt wird.

Bei einer Weiterbildung hat der Klebstoff der Klebstoffschicht, der auf der der ersten Folie zugewandten Seite der zweiten Folie umlaufend um die erste Folie eine Oberfläche der Verbundfolie bilden soll, keinen Kontakt zu einem festen Material, während der Druck auf die Metall-Beschichtung ausgeübt wird. Dadurch wird verhindert, dass der Klebstoff unbeabsichtigt an Gegenständen anhaftet.

Erreicht werden kann dies zum Beispiel durch die im Folgenden beschriebene Ausführungsform des Herstellungsverfahrens: Auf der Seite der Klebstoffschicht wird die erste Folie auf die zweite Folie aufgebracht. Die erste Folie und die zweite Folie werden gemeinsam relativ durch einen Spalt zwischen einer Abstützung der zweiten Folie auf der gegenüberliegenden Seite der Klebstoffschicht und einer Anpressvorrichtung auf der Seite der außenseitigen Metall-Beschichtung der ersten Folie transportiert. Die Spaltdicke, d. h. der Abstand zwischen den einander gegenüberliegenden Oberflächen, die den Anpressdruck ausüben sollen, ist so groß bemessen, dass die vorläufig erzielte Anordnung aus erster und zweiter Folie komprimiert wird und dadurch der Anpressdruck erzielt wird. Andererseits ist die Spaltdicke so klein bemessen, dass der erzielte Folienverbund nicht beschädigt wird und auch kein Kontakt zwischen der Klebstoffschicht und der Anpressvorrichtung entsteht. Dies ist möglich, da die erste Folie eine wenn auch sehr geringe Schichtdicke aufweist. Auch die Art und Dicke der Klebstoffschicht ist dabei zu berücksichtigen. Die Dicke der Klebstoffschicht und die Art des Klebstoffs sind so zu wählen, dass das Anpressen der ersten und der zweiten Folie gegeneinander keine Komprimierung der Klebstoffschicht um mehr als die Dicke des Trägermaterials der ersten Folie erfordert, um den Folienverbund herzustellen. Andernfalls würde ein unerwünschter Kontakt der Anpressvorrichtung zu der Klebstoffschicht hergestellt. Insbesondere kann der Spalt durch eine Walze und einen gegenüberliegenden ruhenden Körper oder durch zwei einander gegenüberliegende Walzen gebildet werden. Die Walze oder die Walzen können derart jeweils um eine Drehachse rotieren, dass ihre rotierende Oberfläche am Außenumfang den Transport der Folie(n) bewirkt oder unterstützt, oder durch den Transport der Folie(n) die Oberfläche am Außenumfang um die Drehachse rotiert wird.

Ausführungsbeispiele der Erfindung werden nun anhand der beigefügten Zeichnung beschrieben. Es zeigen schematisch:
- Fig. 1: eine Draufsicht auf ein erfindungsgemäßes Pflaster,
- Fig. 2: eine überhöhte Seitenansicht des in Fig. 1 dargestellten Pflasters und
- Fig. 3: das in Fig. 2 dargestellte Pflaster bei Zuführung zu einem Spalt zwischen zwei Walzen, die die Folien des Pflasters zu einer Verbundfolie verbinden.

Bei dem als Ausführungsbeispiel gewählten Pflaster 10 handelt es sich um eine Verbundfolie mit einer ersten Folie 1 und einer parallel dazu verlaufenden zweiten Folie 3.

Wie die überhöhte Seitenansicht der Fig. 2 zeigt, weist die erste Folie 1 auf ihrer oben in Fig. 2 liegenden Seite (welche bei Applikation des Pflasters 10 zur Haut des Patienten orientiert ist) eine Metall-Beschichtung 21 auf. Ferner weist die zweite Folie 3 auf ihrer oben in Fig. 2 liegenden Seite eine Klebstoffschicht 23 auf, welche in den Figuren schraffiert dargestellt ist.

Da die erste Folie 1 eine kleinere Breite und eine kleinere Länge als die zweite Folie 3 aufweist und in einem zentralen Bereich der zweiten Folie 3 angeordnet ist, bildet der Klebstoff der Klebstoffschicht 23 eine um die erste Folie 1 umlaufende Oberfläche der Verbundfolie. Die vollflächig an der zweiten Folie 3 ausgebildete Klebstoffschicht 23 dient somit sowohl der Herstellung der Verbundfolie, indem die erste Folie 1 über die Klebstoffschicht 23 mit der zweiten Folie 3 verbunden wird, als auch der Applizierung des Pflasters 10 auf der Haut des Patienten. Der Klebstoff klebt dann an der genannten umlaufenden Oberfläche der Verbundfolie an der Haut des Patienten. An dem mit dem Pflaster zu behandelnden Bereich des Patienten liegt dann die Metall-Beschichtung 21. Nicht dargestellt sind in den Figuren bei Auslieferung des Pflasters 10 eine vorzugsweise vorhandene Schutzschicht (zum Beispiel aus Papier), welche in der Darstellung der Fig. 2 das Pflaster 10 oben abdeckt und insbesondere vollflächig an dem Klebstoff der Klebstoffschicht 23 an der genannten umlaufenden Oberfläche anliegt, und eine vorzugsweise vorhandene Verpackung, die das Pflaster 10 samt Schutzschicht umhüllt.

Die Trägermaterialien der ersten Folie 1 und der zweiten Folie 3 sind in dem Ausführungsbeispiel vorzugsweise jeweils durchsichtige PET-Folien, wobei zumindest eine der Folien 1, 3 einen Füllstoff wie Siliziumdioxid aufweisen kann. Auch kann zumindest eine der beiden Folien 1, 3 durch Ausführung eines Reckverfahrens biaxial ausgestaltet sein. Die Dicke der ersten Folie 1 beträgt beispielsweise überall 12 Mikrometer, die Dicke der zweiten Folie beispielsweise überall 50 Mikrometer. Die Gesamtdicke der Verbundfolie beträgt diesem Fall weniger als 80 Mikrometer. Nicht nur bei der in den Figuren dargestellten Ausführungsform kann die Dicke der zweiten Folie mindestens 20 Mikrometer, insbesondere mindestens 36 Mikrometer und zum Beispiel wie erwähnt 50 Mikrometer betragen. Ferner kann die Dicke der zweiten Folie höchstens 125 Mikrometer, insbesondere höchstens 100 Mikrometer, zum Beispiel höchstens 75 Mikrometer betragen.

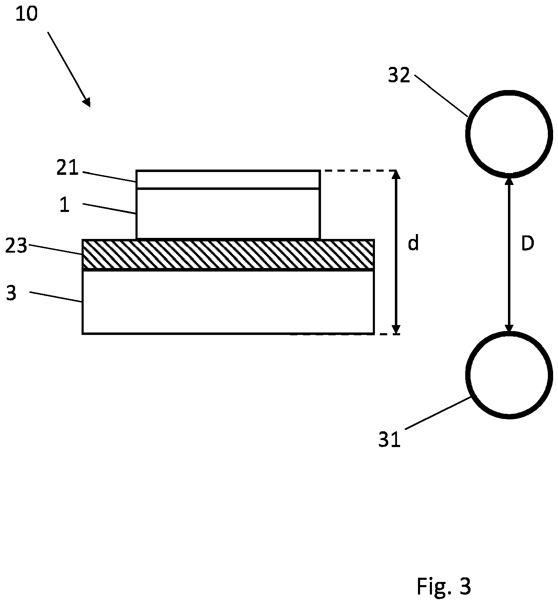

Fig. 3 zeigt schematisch einen Zustand während der Herstellung der Verbundfolie. Vor dem dargestellten Zustand wurde die erste Folie 1 auf der zweiten Folie 3 in der gewünschten Weise zentral positioniert. In dem dargestellten Zustand befindet sich diese Anordnung der beiden Folien 1, 3, welche noch nicht fest miteinander verklebt sind, kurz vor dem Eintritt in einen Spalt mit einer Spaltbreite D. Dieser Spalt wird durch zwei Walzen 31, 32 gebildet. Die Spaltbreite D ist etwas kleiner als die Dicke d der Folien 1, 3 vor ihrer dauerhaft festen Verbindung mittels Anpressen. Das Anpressen wird erreicht, indem die in Fig. 3 dargestellte Anordnung der beiden Folien 1, 3 durch nach rechts durch den Spalt zwischen den beiden Walzen 31, 32 gefördert wird. Da die Spaltbreite D nur etwas kleiner als die Dicke d der beiden Folien 1, 3 ist, kommt die oben liegende Walze 32 nicht in Kontakt mit dem Klebstoff der Klebstoffschicht 23, baut aber dennoch einen Anpressdruck auf die Metall-Beschichtung 21 auf, der zu einer dauerhaften Verbindung der Folien 1, 3 über die Klebstoffschicht 23 führt.

## Patentansprüche

1. Pflaster (10) zur Aufbringung auf die Haut für medizinische, kosmetische und/oder orthopädische Zwecke, wobei
• das Pflaster (10) eine erste Folie (1) aufweist, mit einem ersten Trägermaterial, das auf zumindest einer Seite eine Metall-Beschichtung (21) aufweist,
• das Pflaster (10) eine zweite Folie aufweist, mit einem zweiten Trägermaterial, das auf einer Seite mit einem Klebstoff beschichtet ist, sodass an dem zweiten Trägermaterial eine Klebstoffschicht gebildet ist,
• die erste Folie (1) über die Klebstoffschicht mit der zweiten Folie verbunden ist, sodass eine zweilagige Verbundfolie gebildet ist, bei der eine außenseitig der Verbundfolie liegende Oberfläche durch die Metall-Beschichtung oder eine der Metall-Beschichtungen der ersten Folie gebildet ist, und
• die erste Folie eine kleinere Breite und eine kleinere Länge als die zweite Folie aufweist und derart an der zweiten Folie positioniert ist, dass Klebstoff der Klebstoffschicht auf der der ersten Folie zugewandten Seite der zweiten Folie umlaufend um die erste Folie eine Oberfläche der Verbundfolie bildet.

2. Pflaster nach Anspruch 1, **dadurch gekennzeichnet, dass** die erste Folie (1) einschließlich der Metall-Beschichtung(en) (21) eine Dicke von weniger als 20 µm hat.

3. Pflaster nach einem oder mehreren der vorgenannten Ansprüche, **dadurch gekennzeichnet, dass** das Trägermaterial der ersten Folie (1) und/oder der zweiten Folie ein Polymer ist.

4. Pflaster nach einem oder mehreren der vorgenannten Ansprüche, **dadurch gekennzeichnet, dass** das Trägermaterial der ersten Folie (1) und/oder der zweiten Folie durchsichtig ist.

5. Pflaster nach einem oder mehreren der vorgenannten Ansprüche, **dadurch gekennzeichnet, dass** die Metall-Beschichtung (21), welche die außenseitig der Verbundfolie liegende Oberfläche bildet, undurchsichtig ist.

6. Pflaster nach einem oder mehreren der vorgenannten Ansprüche, **dadurch gekennzeichnet, dass** die Metall-Beschichtung (21), welche die außenseitig der Verbundfolie liegende Oberfläche bildet, Aluminium aufweist.

7. Verfahren zur Herstellung eines Pflasters (10) zur Aufbringung auf die Haut für medizinische, kosmetische und/oder orthopädische Zwecke, insbesondere zur Herstellung des Pflasters (10) nach einem der vorhergehenden Ansprüche, wobei
• eine erste Folie (1) bereitgestellt wird, mit einem ersten Trägermaterial, das auf zumindest einer Seite eine Metall-Beschichtung (21) aufweist,
• eine zweite Folie bereitgestellt wird, mit einem zweiten Trägermaterial, das auf einer Seite mit einem Klebstoff beschichtet ist, sodass an dem zweiten Trägermaterial eine Klebstoffschicht gebildet ist,
• die erste Folie (1) über die Klebstoffschicht mit der zweiten Folie verbunden wird, sodass eine zweilagige Verbundfolie gebildet wird, bei der eine außenseitig der Verbundfolie liegende Oberfläche durch die Metall-Beschichtung oder eine der Metall-Beschichtungen der ersten Folie gebildet wird, und
• die erste Folie eine kleinere Breite und eine kleinere Länge als die zweite Folie aufweist und derart an der zweiten Folie positioniert wird, dass Klebstoff der Klebstoffschicht auf der der ersten Folie zugewandten Seite der zweiten Folie umlaufend um die erste Folie eine Oberfläche der Verbundfolie bildet.

8. Verfahren nach Anspruch 7, **dadurch gekennzeichnet, dass** die erste Folie (1) einschließlich der Metall-Beschichtung(en) (21) eine Dicke von weniger als 20 µm hat.

9. Verfahren nach Anspruch 7 oder 8, **dadurch gekennzeichnet, dass** das Trägermaterial der ersten Folie (1) und/oder der zweiten Folie ein Polymer ist.

10. Verfahren einem der Ansprüche 7 bis 9, **dadurch gekennzeichnet, dass** das Trägermaterial der ersten Folie (1) und/oder der zweiten Folie durchsichtig ist.

11. Verfahren nach einem der Ansprüche 7 bis 10, **dadurch gekennzeichnet, dass** die Metall-Beschichtung (21), welche die außenseitig der Verbundfolie liegende Oberfläche bildet, undurchsichtig ist.

12. Verfahren nach einem der Ansprüche 7 bis 11, **dadurch gekennzeichnet, dass** die Metall-Beschichtung (21), welche die außenseitig der Verbundfolie liegende Oberfläche bildet, Aluminium aufweist.

13. Verfahren nach einem der Ansprüche 7 bis 12, **dadurch gekennzeichnet, dass** die erste Folie (1) durch Druck auf die Metall-Beschichtung (21), welche die außenseitig der Verbundfolie liegende Oberfläche bilden soll, an die Klebstoffschicht der zweiten Folie angepresst wird, um die Verbundfolie zu bilden.

14. Verfahren nach Anspruch 13, **dadurch gekennzeichnet, dass** der Klebstoff der Klebstoffschicht, der auf der der ersten Folie zugewandten Seite der zweiten Folie umlaufend um die erste Folie eine Oberfläche der Verbundfolie bilden soll, keinen Kontakt zu einem festen Material hat, während der Druck auf die Metall-Beschichtung ausgeübt wird.
